# EUROPÄISCHE PATENTSCHRIFT

(11) **EP 1 275 355 B1**
(45) Veröffentlichungstag und Bekanntmachung des Hinweises auf die Patenterteilung: **30.05.2007**
(21) Anmeldenummer: 02012107.5
(22) Anmeldetag: 31.05.2002
(51) Int. Cl.: A61F 2/60, A61F 2/76

(54) **Torsionseinrichtung einer Exoprothese**
Torsion device for exoprosthesis
Dispositif de torsion pour une exoprothèse

(30) Priorität: 29.06.2001 DE 10131159
(43) Veröffentlichungstag der Anmeldung: 15.01.2003
(73) Patentinhaber: Otto Bock HealthCare GmbH, 37115 Duderstadt (DE)
(72) Erfinder: Skiera, Richard, 1210 Wien (AT)
(74) Vertreter: Gramm, Werner

(56) Entgegenhaltungen:
- WO-A-98/29059
- WO-A-98/56320
- DE-A- 19 637 173
- GB-A- 2 305 363

## Beschreibung

Die Erfindung betrifft eine Torsionseinrichtung einer Exoprothese, mit einem proximalen Teil, das entgegen der Wirkung eines federelastischen Torsionselementes relativ gegenüber einem distalen Teil in positiver und negativer Rotationsrichtung verdrehbar ist, wobei das Torsionselement in positiver Rotationsrichtung eine unterschiedliche Torsionscharakteristik gegenüber der in negativer Rotationsrichtung aufweist.

Stoßdämpfer werden in der Exoprothetik zur Abfederung und Dämpfung der Bodenreaktionskräfte eingesetzt. Bodenreaktionskräfte wirken während des Gehens, Laufens oder Springers durch den Bodenkontakt über die Prothese und den Schaft auf den Stumpf und den übrigen Körper. Eine Abfederung und Dämpfung dieser Belastung des empfindlichen Stumpfes und Körpers kann helfen, Stumpfprobleme zu verringern, Überbelastungen des Muskel- und Skelettapparates zu vermeiden und den Komfort für den Patienten zu verbessern. Die Rotationsfähigkeit der Einrichtung gleicht bei Oberschenkelamputierten die fehlende Rotationsfähigkeit des Hüftgelenkes aufgrund der starren Stumpf-Schaft-Verbindung am Becken aus. Dabei kommt der Innenrotation des Fußes gegenüber dem Becken während der Standphase eines Gangzyklus eine besondere Bedeutung zu. Eine erhöhte Rotationssteifigkeit (Rast- oder Initialmoment) für kleine Auslenkungen des Stoßdämpfers werden vom Patienten in Feldversuchen als sehr angenehm und stabilisierend empfunden.

Die eingangs beschriebene Torsionseinrichtung lässt sich der DE 196 37 173 A1 entnehmen. Offenbart ist ein Prothesenträger mit einem darin eingeschlossenen kompressiblen Fluidvolumen zur Abstützung des Gewichts eines Patienten. Dieser Träger umfasst einen ersten an den Beinstumpf des Patienten befestigbaren Endabschnitt sowie einen zweiten, dem ersten Endabschnitt gegenüberliegenden Endabschnitt, der an einer Fußprothese anschließbar ist und gegenüber dem ersten Endabschnitt um eine Längsachse des Trägers drehbar ist. Erster und zweiter Endabschnitt sind über eine Torsionsfeder gekoppelt, die mit einer ersten Federrate einer Drehung in Uhrzeigerrichtung widersteht und einer Drehung entgegen der Uhrzeigerrichtung mit einer zweiten Federrate widersteht, die so ausgewählt ist, dass sie unterschiedlich zur ersten Federrate in Abhängigkeit davon ausgewählt ist, ob der Beinstumpf des Patienten ein rechter oder ein linker Beinstumpf ist.

Aus der GB-A-2 305 363 ist eine gattungsgemäße Torsionseinrichtung einer Exoprothese mit den Merkmalen des Oberbegriffes des Hauptanspruches beschrieben.

Ausgehend von diesem Stand der Technik liegt der Erfindung die Aufgabe zugrunde, die eingangs beschriebene Torsionseinrichtung hinsichtlich ihrer Funktionalität in Bezug auf die Anpassbarkeit der Bedürfnisse eines Nutzers zu verbessern.

Ausgehend von der eingangs beschriebenen Torsionseinrichtung wird diese Aufgabe erfindungsgemäß dadurch gekennzeichnet, dass nur jeweils eines der beiden Torsionsfederelemente in einer der beiden Rotationsrichtungen, nämlich nur in der positiven oder nur in der negativen Rotationsrichtung wirksam ist und dass das Torsionselement in positiver Rotationsrichtung eine unterschiedliche Torsionscharakteristik gegenüber der in negativer Rotationsrichtung aufweist und die Torsionsfederelemente eine unterschiedliche Federcharakteristik aufweisen.

Bei dieser Definition der Erfindung sowie auch in der nachfolgenden Beschreibung könnte das proximale Teil auch ein distales Teil und das distale Teil ein proximales Teil darstellen; und ein Torsionsfederelement könnte auch eine Gruppe von Torsionsfederelementen umfassen.

Mit der Bezeichnung "federelastisch" sollen elastische und/oder viskoelastische und/oder reibungsbehaftete Federelemente sowie eine Viskoplastizität erfasst werden.

In einer speziellen Ausführungsform können die beiden Torsionsfederelemente durch zwei aus einem Elastomer bestehende Ringsegmente gebildet sein, die sich in Rotationsrichtung gesehen direkt oder indirekt jeweils an einem Mitnehmer des proximalen Teils sowie an einem ortsfest am distalen Teil festgelegten Widerlager abstützen.

Zur Erzielung einer kompakten Ausführungsform und einer sicheren Funktion ist es vorteilhaft, wenn die beiden Torsionsfederelemente mit ihrem jeweils ersten Ende an sich in Umfangsrichtung gegenüberliegenden Seiten des Widerlagers und mit ihrem jeweils zweiten Ende an einem jeweils zweiten Widerlager abstützen, wobei diese zweiten Widerlager jeweils in Umfangsrichtung um zumindest einige Umfangsgrad gegen das an ihnen anliegende Torsionsfederelement verschiebbar an dem distalen Teil gelagert sind.

Dabei ist es zur Anpassung der Charakteristik an die Erfordernisse des Patienten zweckmäßig, wenn die Torsionsfederelemente austauschbar sind.

In einer weiteren Ausgestaltung ist es zweckmäßig, wenn die zweiten Widerlager so in Umfangsrichtung verschiebbar gelagert sind, dass jeweils nur eines dieser Widerlager gegen das sich an ihm abstützende Torsionsfederelement verschiebbar ist, während das andere zweite Widerlager durch ein weiteres Widerlager weiterhin unter Vorspannung gehalten bleibt. Diese Lösung führt zu einer ausgeprägten hohen Anfangssteifigkeit.

Erfindungsgemäß lassen sich somit die Rotationseigenschaften der Torsionseinrichtung z.B. bezüglich Innen- und Außenrotation differenziert und individuell anpassen. Die unterschiedlich einstellbaren Torsionssteifigkeiten geben somit die Möglichkeit, eine "weiche" Innenrotation zu ermöglichen und durch eine "harte" Außenrotation während des Fersenstoßes und Zehenabstoßes mehr Stabilität zu erhalten. Wichtig für das Stabilitätsempfinden des Patienten ist dabei, dass bei geringfügigen Torsionsmomenten keine nennenswerte Rotation einsetzt. Die erfindungsgemäß erzielbare drehrichtungsabhängige Wirkung der Torsionsfederelemente erlaubt im Zusammenhang mit ihrer Vorspannung den Aufbau eines ebenfalls drehrichtungsabhängigen Rastmomentes.

Da neben der Belastung durch Torsionsmomente auch Axialkräfte stoßartig auf die Exoprothese und den menschlichen Bewegungsapparat wirken, ist es vorteilhaft, wenn die Torsionseinrichtung mit einem Stoßdämpfersystem kombiniert wird, das ein Axialfederelement aufweist, das sich mit seinem distalen Ende an dem distalen Teil und mit seinem proximalen Ende an dem proximalen, teleskop-artig in den distalen Teil geführten Teil abstützt.

Weitere Merkmale der Erfindung sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen:
- **Figur 1**: eine Torsionseinrichtung im Längsschnitt und
- **Figur 2**: die Torsionseinrichtung gemäß Figur 1 im Querschnitt gemäß der Linie B - B in Figur 1.

Die dargestellte Torsionseinrichtung umfasst ein proximales Teil 1, das teleskopförmig in einem distalen Teil 2 axial geführt ist. Die beiden Teile 1, 2 sind relativ gegeneinander entgegen der Wirkung eines federelastischen Torsionselementes in positiver und negativer Rotationsrichtung verdrehbar. Beim Einsatz dieser Torsionseinrichtung z.B. in einer Unterschenkelprothese kann das proximale Teil 1 mit dem Stumpf eines Prothesenträgers verbindbar sein, während das distale Teil 2 mit einem Prothesenfuß verbunden ist.

In dem dargestellten Ausführungsbeispiel umfasst das Torsionselement zwei separate Torsionsfederelemente 3, 4, die eine unterschiedliche Federcharakteristik aufweisen, und von denen nur jeweils ein Torsionsfederelement in eine der beiden Rotationsrichtungen, nämlich nur in der positiven Rotationsrichtung 5 bzw. nur in der negativen Rotationsrichtung 6 wirksam ist. Dabei sind die beiden Torsionsfederelemente 3, 4 durch zwei aus einem Elastomer bestehende Ringsegmente gebildet, die sich mit ihrem jeweils ersten Ende an sich in Umfangsrichtung gegenüberliegenden Seiten 7a, 7b eines ortsfest am distalen Teil 2 festgelegten Widerlagers 7 abstützen, während sie sich mit ihrem jeweils zweiten Ende an einem jeweils zweiten Widerlager 8, 9 abstützen. Diese zweiten Widerlager 8, 9 sind jeweils in Umfangsrichtung um zumindest einige Umfangsgrad gegen das an ihnen anliegende Torsionsfederelement 3 bzw. 4 verschiebbar an dem distalen Teil 2 gelagert. Die beiden verschiebbaren zweiten Widerlager 8, 9 stützen sich an einem sie bei relativer Rotationsdrehung beaufschlagenden Mitnehmer 10 ab, der am proximalen Teil 1 befestigt ist.

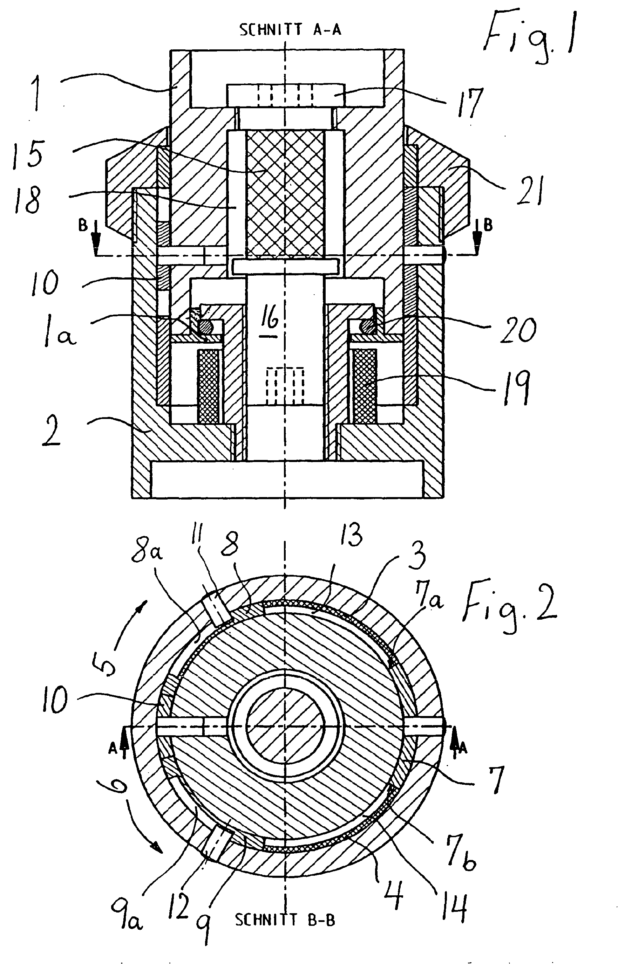

Die Torsionsfederelemente 3, 4 sind unter Vorspannung auf sich gegenüberliegenden Umfangsseiten zwischen die ihnen zugeordneten Widerlager 7, 8 bzw. 7, 9 eingesetzt. Dabei können in einer speziellen Ausführungsform die zweiten Widerlager 8, 9 so in Umfangsrichtung verschiebbar gelagert sein, dass jeweils nur eines dieser Widerlager gegen das sich an ihm abstützende Torsionsfederelement 3 bzw. 4 verschiebbar ist, während das andere zweite Widerlager durch ein weiteres Widerlager 11 bzw. 12 weiterhin unter Vorspannung gehalten bleibt. Diese beiden weiteren Widerlager 11, 12 sind gemäß Figur 2 jeweils als Stift ausgebildet, der fest im distalen Teil 2 angebracht ist und mit einem freien Ende nach innen in eine Verschiebenut 8a bzw. 9a des zweiten Widerlagers 8 bzw. 9 ragt.

Die Torsionsfederelemente 3, 4 sind austauschbar, so dass grundsätzlich auch die Möglichkeit besteht, eines dieser Torsionsfederelemente durch ein weitgehend starres Element zu ersetzen, das dann die Verdrehung der beiden Teile 1, 2 in einer der beiden Rotationsrichtungen 5, 6 quasi blockiert.

Die Torsionsfederelemente 3, 4 bestehen in dem dargestellten Ausführungsbeispiel aus einem weitgehend inkompressiblen Elastomer und beulen bei Torsionsbeaufschlagung durch den Mitnehmer 10 in einen konstruktiv hierfür vorgesehenen Freiraum 13 bzw. 14 aus, der so bemessen sein kann, dass der flache Anstieg der Federkennlinie der Torsionsfederelemente 3, 4 zum Ende der Torsionsdrehung in einen stark progressiven Anstieg übergeht.

Grundsätzlich besteht die Möglichkeit, dass die beiden verschiebbaren Widerlager 8, 9 zugleich Lagerschalen für das proximale Teil 1 bilden, das mit einem rohrförmigen Stutzen in das distale Teil 2 eingeschoben ist.

Der Mitnehmer 10 ist exakt oder mit Übermaß zwischen die beiden verschiebbaren Widerlager 8, 9 eingeschoben.

Die dargestellte Torsionseinrichtung ist auch mit einem Stoßdämpfersystem ausgerüstet, das ein Axialfederelement 15 aufweist, das sich mit seinem distalen Ende auf dem Kopf einer in das proximale Teil 1 eingeschraubten Vorspannschraube 16 und mit seinem proximalen Ende an einer in das proximale Teil 1 eingeschraubten Verschlussschraube 17 abstützt. Durch die Vorspannschraube 16 ist eine Vorspannung stufenlos auf das Axialfederelement 15 aufbringbar. Die Verschlussschraube 17 dient zum Verschluss einer Öffnung, durch die ein Austausch des Axialfederelementes 15 möglich ist.

Das Axialfederelement 15 ist durch einen Elastomerstab gebildet, der aus einem weitgehend inkompressiblen Elastomer besteht und bei Axialbeaufschlagung in einen konstruktiv hierfür vorgesehenen Freiraum ausbeult, der so bemessen sein kann, dass der flache Anstieg der Federkennlinie des Elastomerstabes zum Ende der axialen Einfederung in einen stark progressiven Anstieg übergeht.

Die maximale axiale Einfederung ist durch ein nicht lineares Endanschlagsfederelement 19 begrenzt, das durch einen im distalen Teil 2 festgelegten Elastomerring gebildet ist. Bei axialkraftfreier Torsionseinrichtung liegt das proximale Teil 1 mit einer Ringschulter 1 a an einem den Hub begrenzenden oberen Anschlagsfederelement 20 an. Vorgesehen ist ferner eine Schraubkappe 21, die auf das proximale Ende des distalen Teils 2 aufgeschraubt ist und einen Teil der Teleskopführung für das proximale Teil 1 bildet.

## Patentansprüche

1. Torsionseinrichtung einer Exoprothese, mit einem proximalen Teil (1), das entgegen der Wirkung eines federelastischen Torsionselementes relativ gegenüber einem distalen Teil (2) in positiver und negativer Rotationsrichtung (5, 6) verdrehbar ist, wobei das Torsionselement zwei separate Torsionsfederelemente (3, 4) umfasst, die jeweils unter Vorspannung zwischen ihnen zugeordneten Widerlagern (7, 8; 7, 9) eingesetzt sind, **dadurch gekennzeichnet, dass** die Torsionsfederelemente (3, 4) so zwischen den ihnen zugeordneten Widerlagern (7, 8; 7, 9) angeordnet sind, **dass** nur jeweils eines der beiden Torsionsfederelemente (3, 4) in einer der beiden Rotationsrichtungen (5, 6), nämlich nur in der positiven oder nur in der negativen Rotationsrichtung wirksam ist und **dass** das Torsionselement in positiver Rotationsrichtung (5) eine unterschiedliche Torsionscharakteristik gegenüber der in negativer Rotationsrichtung (6) aufweist.

2. Torsionseinrichtung nach Anspruch 1, **dadurch gekennzeichnet, dass** die beiden Torsionsfederelemente (3, 4) durch zwei aus einem Elastomer bestehende Ringsegmente gebildet sind, die sich in Rotationsrichtung gesehen direkt oder indirekt jeweils an einem Mitnehmer (10) des proximalen Teils (1) sowie an einem ortsfest am distalen Teil (2) festgelegten Widerlager (7) abstützen.

3. Torsionseinrichtung nach Anspruch 2, **dadurch gekennzeichnet, dass** sich die beiden Torsionsfederelemente (3, 4) mit ihrem jeweils ersten Ende an sich in Umfangsrichtung gegenüberliegenden Seiten (7a, 7b) des Widerlagers (7) und mit ihrem jeweils zweiten Ende an einem jeweils zweiten Widerlager (8, 9) abstützen, wobei diese zweiten Widerlager (8, 9) jeweils in Umfangsrichtung um zumindest einige Umfangsgrad gegen das an ihnen anliegende Torsionsfederelement (3, 4) verschiebbar an dem distalen Teil (2) gelagert sind.

4. Torsionseinrichtung nach Anspruch 3, **dadurch gekennzeichnet, dass** die zweiten Widerlager (8, 9) so in Umfangsrichtung verschiebbar gelagert sind, dass jeweils nur eines dieser Widerlager gegen das sich an ihm abstützende Torsionsfederelement (3, 4) verschiebbar ist, während das andere zweite Widerlager durch ein weiteres Widerlager (11, 12) weiterhin unter Vorspannung gehalten bleibt.

5. Torsionseinrichtung nach einem der vorhergehenden Ansprüche, **dadurch gekennzeichnet, dass** die Torsionsfederelemente (3, 4) austauschbar sind.

6. Torsionseinrichtung nach einem der Ansprüche 3 - 5, **dadurch gekennzeichnet, dass** zur Beaufschlagung der beiden verschiebbaren zweiten Widerlager (8, 9) ein am proximalen Teil (1) festgelegter Mitnehmer (10) vorgesehen ist.

7. Torsionseinrichtung nach einem der Ansprüche 2 - 6, **dadurch gekennzeichnet, dass** die Torsionsfederelemente (3, 4) aus einem weitgehend inkompressiblen Elastomer bestehen und bei Torsionsbeaufschlagung durch den Mitnehmer (10) in einen konstruktiv hierfür vorgesehenen Freiraum (13, 14) ausbeulen.

8. Torsionseinrichtung nach Anspruch 7, **dadurch gekennzeichnet, dass** der genannte Freiraum (13, 14) so bemessen ist, **dass** der flache Anstieg der Federkennlinie zum Ende der Torsionsdrehung in einen stark progressiven Anstieg übergeht.

9. Torsionseinrichtung nach einem der Ansprüche 3 - 8, **dadurch gekennzeichnet, dass** die beiden verschiebbaren Widerlager (8, 9) zugleich Lagerschalen für das proximale, mit einem rohrförmigen Stutzen in das distale Teil (2) eingeschobene Teil (1) bilden.

10. Torsionseinrichtung nach einem der vorhergehenden Ansprüche, **dadurch gekennzeichnet, dass** für die beiden Torsionsfederelemente (3, 4) nur ein gemeinsames erstes Widerlager (7) vorgesehen ist.

11. Torsionseinrichtung nach einem der Ansprüche 4 - 10, **dadurch gekennzeichnet, dass** der genannte Mitnehmer (10) exakt oder mit Übermaß zwischen die beiden verschiebbaren Widerlager (8, 9) eingeschoben ist.

12. Torsionseinrichtung nach einem der vorhergehenden Ansprüche, **gekennzeichnet durch** ein Stoßdämpfersystem, das ein Axialfederelement (15) aufweist, das sich mit seinem distalen Ende an dem distalen Teil (2) und mit seinem proximalen Ende an dem proximalen, teleskopartig in dem distalen Teil (2) geführten Teil (1) abstützt.

13. Torsionseinrichtung nach Anspruch 12, **dadurch gekennzeichnet, dass** das Axialfederelement (15) austauschbar eingesetzt ist.

14. Torsionseinrichtung nach Anspruch 12 oder 13, **gekennzeichnet durch** eine Vorspannschraube (16), über die eine Vorspannung stufenlos auf das Axialfederelement (15) aufbringbar ist.

15. Torsionseinrichtung nach Anspruch 12, 13 oder 14, **dadurch gekennzeichnet, dass** das proximale Teil (1) eine über eine Verschlussschraube (17) verschließbare Öffnung für den Austausch des Axialfederelementes (15) aufweist.

16. Torsionseinrichtung nach Anspruch 15, **dadurch gekennzeichnet, dass** die Verschlussschraube (17) zugleich eine Vorspannschraube bildet.

17. Torsionseinrichtung nach einem der Ansprüche 12 - 16, **dadurch gekennzeichnet, dass** die maximale axiale Einfederung durch ein Endanschlagsfederelement (19) begrenzt ist.

18. Torsionseinrichtung nach Anspruch 17, **gekennzeichnet durch** ein nichtlineares Endanschlagsfederelement (19).

19. Torsionseinrichtung nach Anspruch 17 oder 18, **dadurch gekennzeichnet, dass** das Endanschlagsfederelement (19) durch einen im distalen Teil (2) festgelegten Elastomerring gebildet ist.

20. Torsionseinrichtung nach einem der Ansprüche 12 - 19, **dadurch gekennzeichnet, dass** bei axialkraftfreier Torsionseinrichtung das proximale Teil mit einer Ringschulter (1a) an einem den Hub begrenzenden oberen Anschlagsfederelement (20) anliegt.

21. Torsionseinrichtung nach einem der Ansprüche 12 - 20, **gekennzeichnet durch** eine eine Teleskopführung für das proximale Teil (1) bildende Schraubkappe (21), die auf das proximale Ende des distalen Teils (2) aufgeschraubt ist.

22. Torsionseinrichtung nach einem der Ansprüche 12 - 21, **dadurch gekennzeichnet, dass** das Axialfederelement (15) durch einen Elastomerstab gebildet ist.

23. Torsionseinrichtung nach Anspruch 22, **dadurch gekennzeichnet, dass** der Elastomerstab (15) aus einem weitgehend inkompressiblen Elastomer besteht und bei Axialbeaufschlagung in einen konstruktiv hierfür vorgesehenen Freiraum (18) ausbeult, der so bemessen ist, **dass** der flache Anstieg der Federkennlinie zum Ende der axialen Einfederung in einen stark progressiven Anstieg übergeht.

## Claims

1. Torsion device of an exoprosthesis, with a proximal part (1) which, counter to the action of a resilient torsion element, can be turned in positive and negative directions of rotation (5, 6) in relation to a distal part (2), the torsion element comprising two separate torsion spring elements (3, 4) which are each fitted, with prestressing, between associated abutments (7, 8; 7, 9), **characterized in that** the torsion spring elements (3, 4) are arranged between the associated abutments (7, 8; 7, 9) in such a way that in each case only one of the two torsion spring elements (3, 4) is active in one of the two directions of rotation (5, 6), namely only in the positive direction of rotation or only in the negative direction of rotation and **in that** the torsion element in the positive direction of rotation (5) has a torsion characteristic different from that in the negative direction of rotation (6).

2. Torsion device according to Claim 1, **characterized in that** the two torsion spring elements (3, 4) are formed by two ring segments which are made of an elastomer and which, viewed in the direction of rotation, bear directly or indirectly in each case on a carrier (10) of the proximal part (1) and on an abutment (7) fixed in a stationary position on the distal part (2).

3. Torsion device according to Claim 2, **characterized in that** the two torsion spring elements (3, 4) bear with their respective first end on circumferentially opposite sides (7a, 7b) of the abutment (7) and with their respective second end on a respective second abutment (8, 9), these second abutments (8, 9) being mounted on the distal part (2) in such a way that they can each be displaced in the circumferential direction by at least several circumferential degrees relative to the torsion spring element (3, 4) bearing on them.

4. Torsion device according to Claim 3, **characterized in that** the second abutments (8, 9) are mounted so as to be displaceable in the circumferential direction in such a way that in each case only one of these abutments can be displaced relative to the torsion spring element (3, 4) bearing on it, while the other second abutment remains held, with prestressing, by a further abutment (11, 12).

5. Torsion device according to one of the preceding claims, **characterized in that** the torsion spring elements (3, 4) are exchangeable.

6. Torsion device according to one of Claims 3-5, **characterized in that**, in order to act upon the two displaceable second abutments (8, 9), a carrier (10) is provided which is fixed on the proximal part (1).

7. Torsion device according to one of Claims 2-6, **characterized in that** the torsion spring elements (3, 4) are made of a substantially incompressible elastomer and, when subjected to torsion by the carrier (10), buckle into a free space (13, 14) provided for this purpose in the construction.

8. Torsion device according to Claim 7, **characterized in that** said free space (13, 14) is dimensioned such that the shallow rise of the spring characteristic curve merges into a strongly progressive rise at the end of the torsion rotation.

9. Torsion device according to one of Claims 3-8, **characterized in that** the two displaceable abutments (8, 9) at the same time form bearing shells for the proximal part (1) inserted with a tubular attachment piece into the distal part (2).

10. Torsion device according to one of the preceding claims, **characterized in that** only one common first abutment (7) is provided for the two torsion spring elements (3, 4) .

11. Torsion device according to one of Claims 4-10, **characterized in that** said carrier (10) is inserted exactly, or with overdimensioning, between the two displaceable abutments (8, 9).

12. Torsion device according to one of the preceding claims, **characterized by** a shock-absorber system which has an axial spring element (15) which bears with its distal end on the distal part (2) and bears with its proximal end on the proximal part (1) guided telescopically in the distal part (2).

13. Torsion device according to Claim 12, **characterized in that** the axial spring element (15) is inserted in an exchangeable manner.

14. Torsion device according to Claim 12 or 13, **characterized by** a prestressing screw (16) by means of which a prestressing can be applied steplessly to the axial spring element (15).

15. Torsion device according to Claim 12, 13 or 14, **characterized in that** the proximal part (1) has an opening which is used for exchanging the axial spring element (15) and which can be closed off by a closure screw (17).

16. Torsion device according to Claim 15, **characterized in that** the closure screw (17) at the same time forms a prestressing screw.

17. Torsion device according to one of Claims 12-16, **characterized in that** the maximum axial spring compression is limited by an end-stop spring element (19).

18. Torsion device according to Claim 17, **characterized by** a nonlinear end-stop spring element (19).

19. Torsion device according to Claim 17 or 18, **characterized in that** the end-stop spring element (19) is formed by an elastomeric ring fixed in the distal part (2).

20. Torsion device according to one of Claims 12-19, **characterized in that**, with the torsion device free from axial force, the proximal part bears with an annular shoulder (la) on an upper contact spring element (20) which limits the travel.

21. Torsion device according to one of Claims 12-20, **characterized by** a screw cap (21) which forms a telescopic guide for the proximal part (1) and which is screwed onto the proximal end of the distal part (2).

22. Torsion device according to one of Claims 12-21, **characterized in that** the axial spring element (15) is formed by an elastomeric rod.

23. Torsion device according to Claim 22, **characterized in that** the elastomeric rod (15) is made of a substantially incompressible elastomer and, when acted upon axially, buckles into a free space (18) which is provided for this purpose in the construction and which is dimensioned such that the shallow rise of the spring characteristic curve at the end of the axial compression merges into a strongly progressive rise.

## Revendications

1. Dispositif de torsion pour une exoprothèse comprenant une partie proximale (1) qui est mobile en rotation par rapport à une partie distale (2) dans une direction de rotation (5,6) positive et négative à l'encontre de l'effet d'un élément ressort élastique de torsion, dans lequel l'élément de torsion comprend deux éléments ressort de torsion séparés (3,4) qui sont chacun insérés avec précontrainte entre leurs butées (7,8 ; 7,9) correspondantes, **caractérisé en ce que** les éléments de ressort de torsion (3,4) sont disposés entre leurs butées (7,8 ; 7,9) correspondants de telle sorte que seul l'un des deux éléments ressort de torsion (3,4) est efficace dans une des deux directions de rotation (5,6), à savoir seulement dans la direction de rotation positive ou seulement dans la direction de rotation négative et que l'élément de torsion présente dans la direction de rotation positive (5) une caractéristique de torsion différente par rapport à celle qu'elle présente dans la direction de rotation négative.

2. Dispositif de torsion selon la revendication 1, **caractérisé en ce que** les deux éléments ressort de torsion (3,4) sont constitués par deux segments annulaires en élastomère, qui s'appuient chacun, vu dans la direction de rotation directe ou indirecte contre un entraîneur (10) de la partie proximale (1) ainsi que sur une butée (7) fixée de façon fixe en position à la partie distale (2).

3. Dispositif de torsion selon la revendication 2, **caractérisé en ce que** les deux éléments ressort de torsion (3,4) s'appuient chacun par leur première extrémité sur les côtés opposés (7a, 7b) selon la direction périphérique de la butée (7) et chacun par leur seconde extrémité sur une seconde butée (8,9), ces secondes butées (8,9) étant chacune montée de façon mobile sur la partie distale (2) pour pouvoir se déplacer par rapport à l'élément de ressort de torsion (3,4) qui s'appuie sur elle, dans la direction périphérique suivant au moins quelques degrés de cette périphérie.

4. Dispositif de torsion selon la revendication 3, **caractérisé en ce que** les secondes butées (8,9) sont montées mobiles dans la direction périphérique, de façon que seule l'une de ces butées soit mobile vers l'élément ressort de torsion (3,4) qui s'appuie contre elle, tandis que l'autre des deux butées continue à être maintenue sous précontrainte par une autre butée (11,12).

5. Dispositif de torsion selon l'une des revendications précédentes, **caractérisé en ce que** les éléments ressort de torsion (3,4) sont échangeables.

6. Dispositif de torsion selon l'une des revendications 3 à 5, **caractérisé en ce qu'**un entraîneur (10) fixé à la partie proximale (1) est prévu pour entraîner les deux secondes butées mobiles (8,9).

7. Dispositif de torsion selon l'une des revendications 2 à 6, **caractérisé en ce que** les éléments ressort de torsion (3,4) sont constitués par un élastomère sensiblement incompréhensible et que lors de l'entraînement en torsion par l'entraîneur (10), ceux-ci aplanissent un espace libre (13, 14) prévu à cet effet, par construction.

8. Dispositif de torsion selon la revendication 7, **caractérisé en ce que** ledit espace libre (13,14) est dimensionné de telle sorte que l'accroissement plat de la ligne caractéristique du ressort se transforme en un accroissement fortement progressif à la fin de la rotation en torsion.

9. Dispositif de torsion selon l'une des revendications 3 à 8, **caractérisé en ce que** les deux butées mobiles (8,9) forment ensemble des coquilles d'appui pour la partie proximale (1) qui est engagée dans la partie distale (2) avec un embout tubulaire.

10. Dispositif de torsion selon l'une des revendications précédentes, **caractérisé en ce qu'**une seule butée commune (7) est prévue pour les deux éléments ressort de torsion (3,4).

11. Dispositif de torsion selon l'une des revendications 4 à 10, **caractérisé en ce que** ledit entraîneur (10) est engagé exactement ou de façon surdimensionnée entre les deux butées mobiles (8,9).

12. Dispositif de torsion selon l'une des revendications précédentes **caractérisé par** un système d'amortisseur de poussée qui comporte un élément ressort axial, qui s'appuie par son extrémité distale à la partie distale (2) et par son extrémité proximale à la partie proximale (1) qui est guidée téléscopiquement dans la partie distale (2).

13. Dispositif de torsion selon la revendication 12, **caractérisé en ce que** l'élément ressort axial (15) est inséré de façon amovible.

14. Dispositif de torsion selon la revendication 12 ou 13, **caractérisé par** une vis de précontrainte (16) avec laquelle une précontrainte peut être appliquée en continu sur l'élément ressort axial (15).

15. Dispositif de torsion selon la revendication 12, 13 ou 14, **caractérisé en ce que** la partie proximale (1) présente une ouverture pouvant être fermée par une vis de fermeture (17) pour l'échange de l'élément ressort axial (15).

16. Dispositif de torsion selon la revendication 15, **caractérisé en ce que** la vis de fermeture (17) constitue en même temps une vis de précontrainte.

17. Dispositif de torsion selon l'une des revendications 12 à 16, **caractérisé en ce que** la compression maximale du ressort est limitée par une butée de fin de course (19) formant ressort.

18. Dispositif de torsion selon la revendication 17, **caractérisé par** une butée de fin de course (19) formant ressort non linéaire.

19. Dispositif de torsion selon la revendication 17 ou 18, **caractérisé en ce que** la butée de fin de course (19) formant ressort est constituée par un anneau en élastomère fixé à la partie distale (2).

20. Dispositif de torsion selon l'une des revendications 12 à 19, **caractérisé en ce que** dans le cas d'un dispositif de torsion sans effort axial, la partie proximale s'appuie au moyen d'un épaulement annulaire (la) contre la butée supérieure de l'élément ressort (20) qui limite la course.

21. Dispositif de torsion selon l'une des revendications 12 à 20, **caractérisé par** un capuchon vissé (21) pour le guidage téléscopique de la partie proximale (1) qui est vissé sur l'extrémité proximale de la partie distale (2).

22. Dispositif de torsion selon l'une des revendications 12 à 21, **caractérisé en ce que** l'élément ressort axial (15) est constitué par une tige en élastomère.

23. Dispositif de torsion selon la revendication 22, **caractérisé en ce que** la tige en élastomère (15) est en élastomère sensiblement incompressible qui en cas d'appui axial aplanit un espace libre (18) construit à cet effet, qui est dimensionné de telle sorte que l'accroissement plat de la ligne de caractéristique du ressort se transforme en fin de compression axiale en un accroissement fortement progressif.
